# EUROPÄISCHE PATENTSCHRIFT

(11) **EP 1 637 066 B1**
(45) Veröffentlichungstag und Bekanntmachung des Hinweises auf die Patenterteilung: **02.12.2009**
(21) Anmeldenummer: 05020050.0
(22) Anmeldetag: 15.09.2005
(51) Int. Cl.: A61B 1/267

(54) **Spreizbares medizinisches Instrument für endoskopische Eingriffe**
Spreadable medical instrument for endoscopic surgery
Instrument médical écartable pour chirurgie endoscopique

(30) Priorität: 20.09.2004 DE 102004045502
(43) Veröffentlichungstag der Anmeldung: 22.03.2006
(73) Patentinhaber: Karl Storz GmbH & Co. KG, 78532 Tuttlingen (DE)
(72) Erfinder: Efinger, Andreas, 78604 Rietheim (DE); Hermle, Rainer, 78559 Gosheim (DE)
(74) Vertreter: Hofmeister, Frank

(56) Entgegenhaltungen:
- US-A- 3 332 414
- US-A- 3 847 143
- US-A- 5 092 314
- US-A- 5 938 591
- US-A1- 2002 165 433
- US-B1- 6 416 467
- US-B1- 6 494 828

## Beschreibung

Die Erfindung betrifft ein spreizbares medizinisches Instrument für endoskopische Eingriffe mit einem Grundkörper, einem an dem Grundköper angeordneten Handgriff und mindestens zwei mit dem Handgriff verbundenen Spatelblättern, die über einen Einstellmechanismus zwischen einer geschlossenen Ausgangsstellung und wenigstens einer Arbeitsstellung parallel und winklig gegeneinander verstellbar sind, wobei der Einstellmechanismus derart am Handgriff angeordnet ist, dass das Ergreifen des Handgriffs und das Betätigen des Einstellmechanismus gleichzeitig mit derselben Hand erfolgen kann.

Derartige spreizbare Instrumente für endoskopische Eingriffe sind in verschiedenen Ausführungsformen bekannt. Ein bevorzugter Anwendungsfall ist die Ausbildung eines solchen Instruments als Laryngoskop. Laryngoskope werden insbesondere zur direkten instrumentellen Inspektion des Kehlkopfes sowie für endolarygeale chirurgische Eingriffe verwendet. Durch die Verstellbarkeit der Spatelblätter ist es möglich, das durch die einander zugewandten, im Querschnitt im wesentlichen halbkreisförmig ausgebildeten Spatelblätter gebildete freie Lumen so zu variieren, dass das Laryngoskop in einer geschlossenen Ausgangsstellung mit einem minimalen Lumen in den Mund- und Rachenraum des intubierten und narkotisierten Patienten eingeführt wird und erst nachträglich das Lumen durch paralleles und/oder winkliges Verstellen der Spatelblätter zueinander so vergrößert wird, dass geeignete medizinische Instrumente in das Laryngoskop einsetzbar sind.

Weitere Anwendungsgebiete für derartige spreizbare medizinische Instrumente sind insbesondere die "neck-surgery" sowie die Verwendung als Mediastinoskop.

Ein gattungsgemäßes spreizbares medizinisches Instrument ist beispielsweise aus der US-A-5 938 591 bekannt. Diese Druckschrift offenbart ein Laryngoskop mit zwei Spatelblättern, die über jeweils einen Rasthebelmechanismus parallel und winklig gegeneinander verstellbar sind. Die Rasthebelmechanismen sind dabei so ausgebildet, dass diese bei Betätigung die Spatelblätter in vorgegebenen Raststellung zueinander verrasten. Neben der ausladenden Bauweise der weit vom Handgriff abstehenden Rasthebel weist diese bekannte Konstruktion den Nachteil auf, dass die Spatelblätter nur in den vorgegebenen Rastschritten zueinander verstellbar sind, was in der Praxis zu einer für den Bedarfsfall zu engen oder aber zu weiten Stellung der Spatelblätter führen kann.

Ein weiteres, als Laryngoskop ausgebildetes Instrument ist beispielsweise aus der DE 199 54 442 A1 bekannt. Bei diesem bekannten Laryngoskop sind der Handgriff und die Stellelemente des Einstellmechanismus zum Verstellen der Spatelblätter so angeordnet, dass der Operateur hierzu beide Hände benutzen muss, was bei der Laryngoskopie nicht besonders nachteilig ist, da das Laryngoskop nach der Positionierung patienten- oder OP-Tisch-fest fixiert wird.

Für den Einsatz bei der "neck-surgery" beispielsweise, wird aber ein Endoskop mit Spateln in eine Inzision im Nacken eingeführt und die Spatel dann das Gewebe dehnend soweit auseinander bewegt, bis ein ausreichender Zugang zum Operationsgebiet geschaffen ist. Für diese Operationstechnik sowie auch bei der Verwendung als Mediastinoskop ist es unumgänglich, dass der Operateur eine Hand zur Bedienung weiterer medizinischer Instrumente frei hat.

Davon ausgehend liegt der Erfindung die **Aufgabe** zugrunde, ein spreizbares medizinisches Instrument für endoskopische Eingriffe so auszugestalten, dass dieses möglichst vielseitig einsetzbar und einfach zu bedienen ist.

Die **Lösung** dieser Aufgabenstellung ist erfindungsgemäß dadurch gekennzeichnet, dass der Einstellmechanismus als stufenlos verstellbarer Schraubmechanismus ausgebildet ist.

Durch die erfindungsgemäße Ausgestaltung ist es erstmalig möglich, ein spreizbares medizinisches Instrument für endoskopische Eingriffe mit einer Hand zu ergreifen und gleichzeitig mit derselben Hand den stufenlosen Schraubmechanismus zum Verstellen der Spatelblätter zu betätigen, wobei beispielsweise der Operateur mit der Hand, mit der er den Handgriff umgreift, mit einem oder mehreren Fingern an den Schraubmechanismus reichen und diesen betätigen kann.

Aufgrund dieser Einhandbedienbarkeit hat der Operateur stets eine Hand frei, um ein weiteres medizinisches Instrument bedienen zu können. Somit eignet sich ein solchermaßen ausgebildetes spreizbares medizinisches Instrument zusätzlich zur Verwendung als Laryngoskop auch für andere endoskopische Eingriffe und Verwendungszwecke.

Die Einsatzmöglichkeit des erfindungsgemäßen Instruments lässt sich dadurch erweitern, dass der Einstellmechanismus zum Verstellen der Spatelblätter eine Stellschraube zur Parallelverstellung wenigstens eines der Spatelblätter sowie eine Stellschraube zur Kippverstellung wenigstens eines der Spatelblätter umfasst, so dass die Spatelblätter unabhängig voneinander stufenlos entweder verschoben oder verkippt werden können.

Um die Spatelblätter in der eingestellten Position zu halten, wird mit der Erfindung weiterhin vorgeschlagen, dass die Stellschrauben selbsthemmend ausgebildet sind.

Gemäß einer praktischen Ausführungsform der Erfindung wird vorgeschlagen, dass über die Stellschraube zur Kippverstellung des wenigstens einen Spatelblatts zusätzlich oder gleichzeitig der Handgriff gegenüber dem Grundkörper verkippbar ist. Die Verkippbarkeit des Handgriffs gibt dem Operateur eine weitere Möglichkeit, das medizinische Instrument in eine definierte stabile Lage zu überführen.

Um zu ermöglichen, dass die Spatelblätter auch dann gegeneinander verkippbar sind, wenn die Spatelblätter ausgehend von der aneinander anliegenden Ausgangsstellung nicht vorher parallel zueinander verschoben wurden, wird erfindungsgemäß vorgeschlagen, dass zumindest ein Spatelblatt am proximalen Ende eine Abschrägung aufweist, die derart ausgebildet ist, dass in der Ausgangsstellung mit aneinander anliegenden, geschlossenen Spatelblättern zwischen diesen sich zum proximalen Ende hin ein Spalt öffnet.

Diese an zumindest einem Spatelblatt ausgebildete Abschrägung gewährleistet, dass die Spatelblätter parallelverschiebungsfrei um einen vorwählbaren Winkel, vorzugsweise 7°, gegeneinander verkippbar sind.

Um weiterhin zu verhindern, dass bei annähernd geschlossener Stellung der Spatelblätter zueinander Gewebe zwischen die Spatelblättern eindringen kann und dort möglicherweise eingeklemmt wird, wird mit der Erfindung vorgeschlagen, dass die Kontaktlinie, entlang der die beiden Spatelblätter in der geschlossenen Ausgangsstellung aneinander anliegen, im Bereich des distalen Endes in Form mindestens einer Stufe ausgebildet ist, die sich in proximaler Richtung erhöht oder absenkt. Diese Stufenform verhindert in der annähernd geschlossenen Stellung der Spatelblätter das Ausbilden eines geradlinig durchgehenden Spalts zwischen den Spatelblättern, in den Gewebe eintreten kann.

Weiterhin wird mit der Erfindung vorgeschlagen, dass die Kontaktflächen entlang derer die beiden Spatelblätter in der geschlossenen Ausgangsstellung aneinander anliegen selbstzentrierend ausgebildet sind, wodurch ein Verkanten und/oder gegenseitiges Unter- bzw. Übergreifen der Spatelblätter speziell beim Schließvorgang der Spatelblätter zueinander verhindert werden kann. Gemäß einer bevorzugten Ausführungsform der Erfindung wird diese Selbstzentrierung dadurch erzielt, dass die Kontaktflächen der Spatelblätter gegenseitig korrespondierend schrägwinklig zur Mittelachse der Spatelblätter ausgebildet sind.

Um das erfindungsgemäße medizinische Instrument einfach und vollständig reinigen zu können, ist eine mit diesem Instrument verwendbare endoskopische Kamera auswechselbar in den Grundkörper des Instruments einsetzbar.

Schließlich wird mit der Erfindung vorgeschlagen, dass ein Spatelblatt eine Führungshülse zur Aufnahme einer Kameraoptik aufweist. Die Verwendung dieser Führungshülse erleichtert einerseits das Einsetzen der Kameraoptik in das Instrument und bewirkt andererseits eine exakte Positionierung der Optik bei gleichzeitigem Schutz vor Beschädigungen.

Weitere Merkmale und Vorteile der Erfindung ergeben sich anhand der zugehörigen Zeichnung, in der zwei Ausführungsbeispiele eines erfindungsgemäßen spreizbaren medizinischen Instruments für endoskopische Eingriffe nur beispielhaft dargestellt sind. In der Zeichnung zeigt:
- Fig. 1: eine teilweise geschnittene Seitenansicht einer ersten Ausführungsform eines erfindungsgemäßen spreizbaren medizinischen Instruments im geschlossenen Zustand;
- Fig. 2: eine Schnittdarstellung gemäß Fig. 1, jedoch das medizinische Instrument in einer geöffneten Arbeitsstellung darstellend;
- Fig. 3: eine Schnittdarstellung gemäß Fig. 2, jedoch das medizinische Instrument zusätzlich mit verkipptem Handgriff darstellend;
- Fig. 4: einen vergrößerten Schnitt entlang der Linie IV-IV gemäß Fig. 1 und
- Fig. 5: eine ausschnittweise schematische Seitenansicht einer zweiten Ausführungsform zur Ausgestaltung der Spatelblätter.

Das in den Abbildungen dargestellte spreizbare medizinische Instrument besteht im wesentlichen aus einem mit einem Handgriff 1 versehenen Grundkörper 2 und zwei mit dem Handgriff 1 verbundenen Spatelblättern 3 und 4, die über einen Einstellmechanismus 5 gegeneinander verstellbar sind.

Ein solches medizinisches Instrument ist beispielsweise als Laryngoskop oder Mediastinoskop sowie als Instrument für die endoskopische "neck-surgery" verwendbar. Entscheidendes Merkmal dieser spreizbaren medizinischen Instrumente ist, dass die Spatelblätter 3, 4 zwischen einer geschlossenen Ausgangsstellung und wenigstens einer gespreizten Arbeitsstellung verstellbar sind, in der die Spatelblätter 3, 4 parallel und/oder winklig gegeneinander verstellt sind.

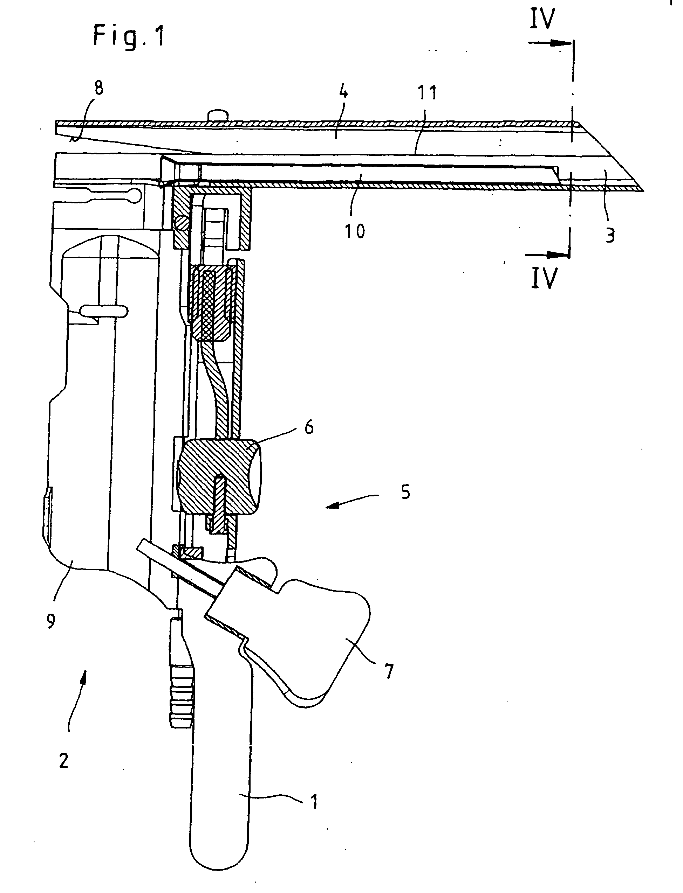

Abbildung Fig. 1 zeigt das Instrument in der geschlossenen Ausgangsstellung, in der die Spatelblätter 3, 4 fest aufeinander anliegen, um den Spatelteil des Instruments mit einem minimalen Lumen in das Operationsgebiet, beispielsweise in den Mund- und Rachenraum bei einem Laryngoskop, einführen und aus diesem wieder entfernen zu können. Das verbleibende Lumen ist ausreichend, um mittels eines an- oder eingesetzten Lichtträgers die Lage des Instruments im Operationsgebiet zu bestimmen.

Aufgrund der Verstellbarkeit der Spatelblätter 3 und 4 ist es möglich, das durch die einander zugewandten, im Querschnitt im wesentlichen halbkreisförmig ausgebildeten Spatelblätter 3, 4 gebildete freie Lumen so zu variieren, dass dieses ausgehend von der geschlossenen Ausgangsstellung nachträglich durch paralleles und/oder winkliges Verstellen der Spatelblätter 3, 4 zueinander so vergrößert wird, dass geeignete medizinische Instrumente in die gespreizten Spatelblätter 3, 4 einsetzbar sind.

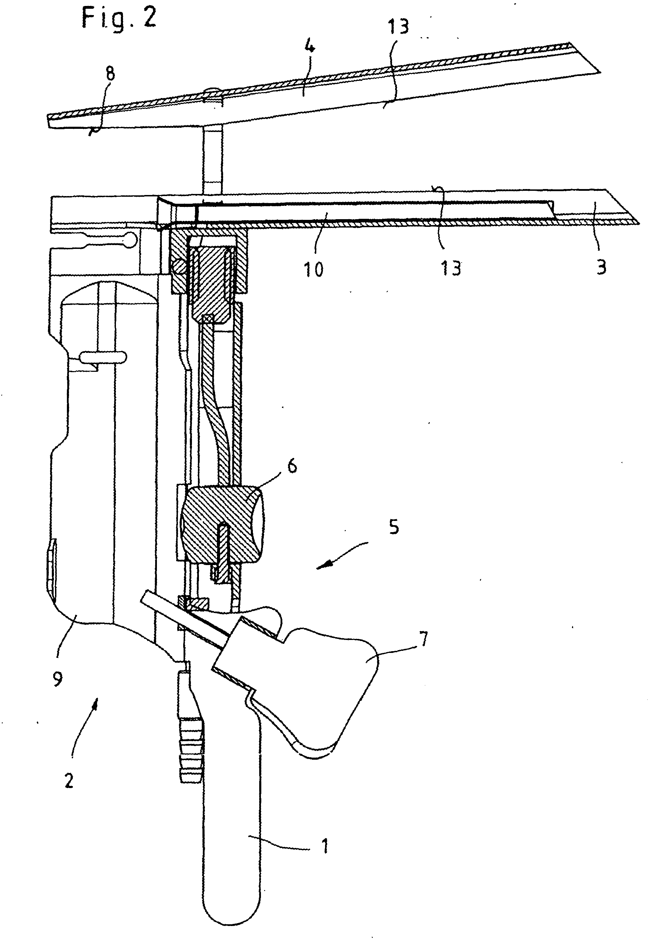

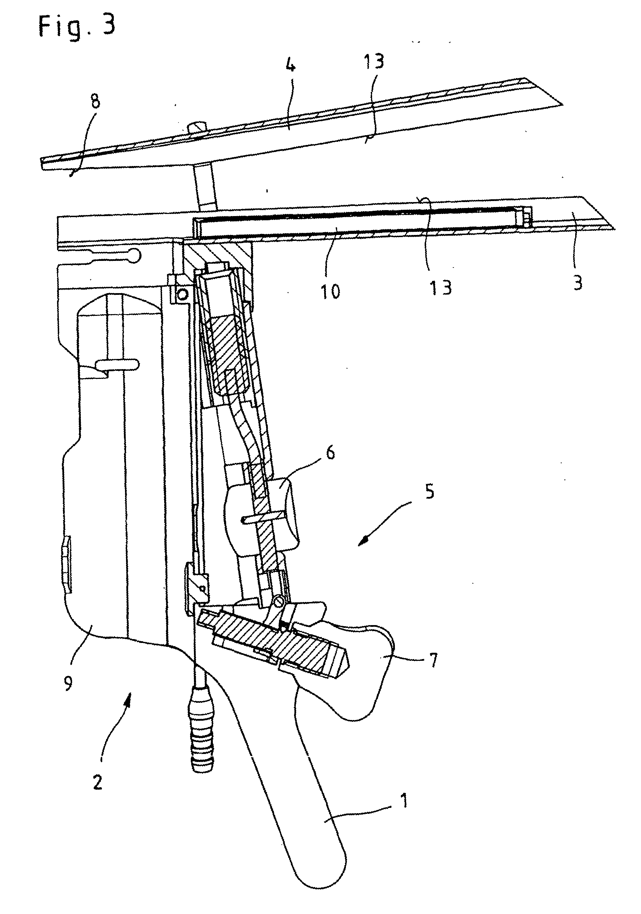

Wie aus den Abbildungen Fig. 1 bis Fig. 3 ersichtlich, besteht der Einstellmechanismus 5 zum Verstellen der Spatelblätter 3, 4 bei der dargestellten Ausführungsform aus zwei separaten stufenlos verstellbaren Stellschrauben 6 und 7, wobei die Betätigung der Stellschraube 6 eine Parallelverschiebung des Spatelblatts 4 gegenüber dem Spatelblatt 3 bewirkt und über die Stellschraube 7 das Spatelblatt 4 gegenüber dem Spatelblatt 3 verkippbar ist, wie dies den Abbildungen Fig. 2 und Fig. 3 zu entnehmen ist. Fig. 3 zeigt weiterhin, dass zusätzlich auch der Handgriff 1 über die Stellschraube 7 gegenüber dem Grundkörper 2 verkippbar ausgebildet sein kann, um dem Operateur eine jederzeit passende und sichere Arbeitshaltung zu ermöglichen.

Selbstverständlich ist es auch möglich, die Stellschrauben 6 und 7 in einer einzigen Stellschraube zusammen zu fassen, über welche dann, beispielsweise durch verschiedene Drehrichtungen, beide Verstellmöglichkeiten, nämlich Parallelverschiebung und Verkippen, gezielt und stufenlos ausführbar sind.

Wie weiterhin aus den Abbildungen Fig. 1 bis Fig. 3 ersichtlich ist, sind die Stellschrauben 6 und 7 des Einstellmechanismus 5 so am Handgriff 1 angeordnet, dass das Ergreifen des Handgriffs 1 und das Betätigen des Einstellmechanismus 5 gleichzeitig mit derselben Hand erfolgen kann, wobei beispielsweise der Operateur mit der Hand, mit der er den Handgriff 1 umgreift, mit einem oder mehreren Fingern an den Einstellmechanismus 5 reichen und diesen betätigen kann.

Um zu ermöglichen, dass die Spatelblätter auch dann gegeneinander verkippbar sind, wenn die Spatelblätter 3, 4 auch ausgehend von der aneinander anliegenden Ausgangsstellung nicht vorher parallel zueinander verschoben wurden, weist bei der dargestellten Ausführungsform das Spatelblatt 4 am proximalen Ende eine Abschrägung 8 auf, die derart ausgebildet ist, dass in der aneinander anliegenden geschlossenen Ausgangsstellung der Spatelblätter 3, 4 ein zum proximalen Ende hin offener Spalt zwischen den Spatelblättern 3, 4 ausgebildet ist. Selbstverständlich ist es auch möglich, die Abschrägung 8 nur am Spatelblatt 3, oder aber an beiden Spatelblättern 3, 4 auszubilden.

Die Abschrägung 8, die gleichzeitig einen Anschlag für den maximalen Verkippwinkel darstellt, ist durch Auswahl der entsprechenden Spatelblätter 3, 4 dem jeweiligen Anwendungsfall entsprechend vor dem Einsatz des Instruments auswählbar und festlegbar. Bei der Laryngoskopie beispielsweise beträgt dieser Winkel vorzugsweise 7°.

Der Grundkörper 2 des medizinischen Instruments besteht im wesentlichen aus einem zu öffnenden und wieder verschließbaren Kamerafach 9, das zur auswechselbaren Aufnahme einer endoskopischen Kamera dient. Im Gegensatz zu den aus dem Stand der Technik bekannten fest eingebauten Kameras hat die Auswechselbarkeit den Vorteil, dass das gesamte Instrument einfach und vollständig zu reinigen ist, beispielsweise zu autoklavieren. Zur Aufnahme der Kameraoptik ist auf der Innenseite des Spatelblatts 3 eine Führungshülse 10 angeordnet. Die Verwendung dieser Führungshülse 10 erleichtert einerseits das Einsetzen der Kameraoptik in das Instrument und bewirkt andererseits eine exakte Positionierung der Optik bei gleichzeitigem Schutz vor Beschädigungen.

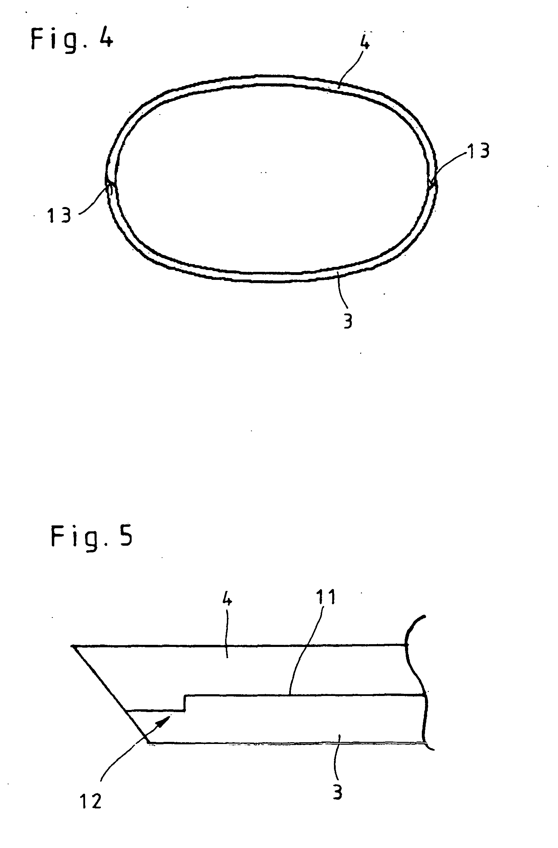

Um zu verhindern, dass bei annähernd geschlossener Stellung der Spatelblätter 3, 4 Gewebe zwischen die Spatelblättern 3, 4 eindringen kann und dort möglicherweise eingeklemmt wird, ist die Kontaktlinie 11, entlang der die beiden Spatelblätter 3, 4 in der geschlossenen Ausgangsstellung aneinander anliegen, im Bereich des distalen Endes in Form mindestens einer Stufe 12 ausgebildet, wie dies der in Fig. 5 dargestellten Ausführungsform zu entnehmen ist. Diese Stufenform verhindert in der annähernd geschlossenen Stellung der Spatelblätter 3, 4 das Ausbilden eines geradlinig durchgehenden Spalts zwischen den Spatelblättern 3, 4, in den Gewebe eintreten kann.

Die Abbildung Fig. 4 zeigt schließlich eine besondere Ausgestaltungsform der Kontaktflächen 13, entlang derer die beiden Spatelblätter 3, 4 in der geschlossenen Ausgangsstellung aneinander anliegen. Wie aus der Abbildung ersichtlich, sind die Kontaktflächen 13 der Spatelblätter 3, 4 gegenseitig korrespondierend schrägwinklig zur Mittelachse der Spatelblätter 3, 4 ausgebildet. Diese schrägwinklige Ausgestaltung bewirkt eine Selbstzentrierung der Spatelblätter 3, 4 in der geschlossenen Stellung, wodurch ein Verkanten, Verdrehen und/oder gegenseitiges Unter- bzw. Übergreifen der Spatelblätter 3, 4 verhindert wird.

Ein solchermaßen ausgebildetes spreizbares medizinisches Instrument zeichnet sich dadurch aus, dass es aufgrund der Einhandbedienbarkeit vielseitig einsetzbar und einfach bedienbar ist.

### B e z u a s z e i c h e n l i s t e

- 1: Handgriff
- 2: Grundkörper
- 3: Spatelblatt
- 4: Spatelblatt
- 5: Einstellmechanismus
- 6: Stellschraube
- 7: Stellschraube
- 8: Abschrägung
- 9: Kamerafach
- 10: Führungshülse
- 11: Kontaktlinie
- 12: Stufe
- 13: Kontaktfläche

## Patentansprüche

1. Spreizbares medizinisches Instrument für endoskopische Eingriffe mit einem Grundkörper (2), einem an dem Grundköper (2) angeordneten Handgriff (1) und mindestens zwei mit dem Handgriff (1) verbundenen Spatelblättern (3, 4), die über einen Einstellmechanismus (5) zwischen einer geschlossenen Ausgangsstellung und wenigstens einer Arbeitsstellung sowohl parallel zueinander als auch winklig gegeneinander verstellbar sind, **dadurch gekennzeichnet, dass** der Einstellmechanismus (5) derart am Handgriff (1) angeordnet ist, dass das Ergreifen des Handgriffs (1) und das Betätigen des Einstellmechanismus (5) gleichzeitig mit derselben Hand erfolgen kann
und der Einstellmechanismus (5) als stufenlos verstellbarer Schraubmechanismus ausgebildet ist.

2. Spreizbares medizinisches Instrument nach Anspruch 1, **dadurch gekennzeichnet, dass** der Einstellmechanismus (5) eine Stellschraube (6) zur Parallelverstellung wenigstens eines der Spatelblätter (3) sowie eine Stellschraube (7) zur Kippverstellung wenigstens eines der Spatelblätter (4) umfasst.

3. Spreizbares medizinisches Instrument nach Anspruch 2, **dadurch gekennzeichnet, dass** die Stellschrauben (6, 7) selbsthemmend ausgebildet sind.

4. Spreizbares medizinisches Instrument nach einem der Ansprüche 1 bis 3, **dadurch gekennzeichnet, dass** der Handgriff (1) gegenüber dem Grundkörper (2) verkippbar ist.

5. Spreizbares medizinisches Instrument nach Anspruch 4, **dadurch gekennzeichnet, dass** über die Stellschraube (7) zur Kippverstellung des wenigstens einen Spatelblatts (4) zusätzlich oder gleichzeitig der Handgriff (1) gegenüber dem Grundkörper (2) verkippbar ist.

6. Spreizbares medizinisches Instrument nach einem der Ansprüche 1 bis 5, **dadurch gekennzeichnet, dass** zumindest ein Spatelblatt (4) am proximalen Ende eine Abschrägung (8) aufweist, die derart ausgebildet ist, dass in der Ausgangsstellung mit aneinander anliegenden, geschlossenen Spatelblättern (3, 4) zwischen den Spatelblättern (3, 4) sich zum proximalen Ende hin ein Spalt öffnet.

7. Spreizbares medizinisches Instrument nach Anspruch 6, **dadurch gekennzeichnet, dass** über die an zumindest einem Spatelblatt (4) ausgebildete Abschrägung (8) die Spatelblätter (3, 4) parallelverschiebungsfrei um einen vorwählbaren Winkel, vorzugsweise 7°, gegeneinander verkippbar sind.

8. Spreizbares medizinisches Instrument nach einem der Ansprüche 1 bis 7, **dadurch gekennzeichnet, dass** in den Grundkörper (2) auswechselbar eine endoskopische Kamera einsetzbar ist.

9. Spreizbares medizinisches Instrument Anspruch 8, **dadurch gekennzeichnet, dass** ein Spatelblatt (3) eine Führungshülse (10) zur Aufnahme einer Kameraoptik aufweist.

## Claims

1. Spreadable medical instrument for endoscopic interventions, with a main body (2), a handle (1) arranged on the main body (2), and at least two spatula blades (3, 4) that are connected to the handle (1) and that can be moved between a closed starting position and at least one working position, both parallel to each other and also at an angle to each other, by an adjustment mechanism (5), **characterized in that** the adjustment mechanism (5) is arranged on the handle (1) in such a way that it is possible simultaneously to grip the handle (1) and actuate the adjustment mechanism (5) using the same hand, and the adjustment mechanism (5) is designed as a continuously adjustable screw mechanism.

2. Spreadable medical instrument according to Claim 1, **characterized in that** the adjustment mechanism (5) comprises a regulating screw (6) for the parallel adjustment of at least one of the spatula blades (3), and also a regulating screw (7) for adjusting at least one of the spatula blades (4) by tilting it.

3. Spreadable medical instrument according to Claim 2, **characterized in that** the regulating screws (6, 7) are self-locking.

4. Spreadable medical instrument according to one of Claims 1 to 3, **characterized in that** the handle (1) can be tilted relative to the main body (2).

5. Spreadable medical instrument according to Claim 4, **characterized in that** the regulating screw (7) for adjusting the at least one spatula blade (4) by tilting it can additionally or simultaneously tilt the handle (1) relative to the main body (2).

6. Spreadable medical instrument according to one of Claims 1 to 5, **characterized in that** at least one spatula blade (4) has, at the proximal end, a bevel (8) which is designed in such a way that, in the starting position with closed spatula blades (3, 4) resting on each other, a gap extending towards the proximal end opens up between the spatula blades (3, 4).

7. Spreadable medical instrument according to Claim 6, **characterized in that**, by means of the bevel (8) formed on at least one spatula blade (4), the spatula blades (3, 4) can be tilted relative to each other by a predefinable angle, preferably 7°, without parallel movement.

8. Spreadable medical instrument according to one of Claims 1 to 7, **characterized in that** an endoscopic camera can be inserted exchangeably into the main body (2).

9. Spreadable medical instrument according to Claim 8, **characterized in that** one spatula blade (3) has a guide sleeve (10) for receiving a camera lens.

## Revendications

1. Instrument médical expansible par écartement, destiné à des interventions endoscopiques, comprenant un corps de base (2), une poignée (1) agencée sur le corps de base (2), et au moins deux lames de spatule (3, 4) reliées à la poignée (1) et qui, à l'aide d'un mécanisme de réglage (5), peuvent être déplacées ou réglées aussi bien parallèlement l'une à l'autre que de manière angulaire l'une par rapport à l'autre, entre une position initiale fermée et au moins une position de travail,
**caractérisé en ce que** le mécanisme de réglage (5) est agencé sur la poignée (1) de manière à ce que la saisie de la poignée (1) et l'actionnement du mécanisme de réglage (5) puissent s'effectuer simultanément avec la même main, et le mécanisme de réglage (5) est réalisé sous la forme d'un mécanisme à vis réglable en continu.

2. Instrument médical expansible par écartement selon la revendication 1, **caractérisé en ce que** le mécanisme de réglage (5) comprend une vis de réglage (6) pour le déplacement parallèle d'au moins une des lames de spatule (3), ainsi qu'une vis de réglage (7) pour le déplacement par basculement de l'une des lames de spatule (4).

3. Instrument médical expansible par écartement selon la revendication 2, **caractérisé en ce que** les vis de réglage (6, 7) sont de conception autobloquante.

4. Instrument médical expansible par écartement selon l'une des revendications 1 à 3, **caractérisé en ce qu'**il est possible de faire basculer la poignée (1) par rapport au corps de base (2).

5. Instrument médical expansible par écartement selon la revendication 4, **caractérisé en ce que** par l'intermédiaire de la vis de réglage (7) pour le déplacement par basculement d'au moins une lame de spatule (4), il est possible de faire basculer en plus ou simultanément la poignée (1) par rapport au corps de base (2).

6. Instrument médical expansible par écartement selon l'une des revendications 1 à 5, **caractérisé en ce qu'**au moins une lame de spatule (4) présente à l'extrémité proximale, un biseau (8) conçu de façon telle que dans la position initiale dans laquelle les lames de spatule (3, 4) sont fermées et appliquées l'une contre l'autre, une fente s'ouvre entre les lames de spatule (3, 4), en direction de l'extrémité proximale.

7. Instrument médical expansible par écartement selon la revendication 6, **caractérisé en ce que** par l'intermédiaire du biseau (8) formé sur l'une au moins des lames de spatule (4), il est possible de faire basculer les lames de spatule (3, 4) l'une par rapport à l'autre, sans coulissement parallèle, d'un angle pouvant être prédéterminé, de préférence d'une valeur de 7°.

8. Instrument médical expansible par écartement selon l'une des revendications 1 à 7, **caractérisé en ce qu'**il est possible d'insérer de manière interchangeable une caméra endoscopique dans le corps de base (2).

9. Instrument médical expansible par écartement selon la revendication 8, **caractérisé en ce qu'**une lame de spatule (3) comporte une douille de guidage (10) destinée à recevoir une optique de caméra.
